# EUROPEAN PATENT APPLICATION

(11) **EP 1 202 210 A2**
(43) Date of publication of application: **02.05.2002**
(21) Application number: 01308918.0
(22) Date of filing: 19.10.2001
(51) Int. Cl.: G06F 19/00

(54) **Medical software methods and systems**

(30) Priority: 31.10.2000 JP 2000332215
(71) Applicant: GE Medical Systems Global Technology Company LLC, Waukesha, Wisconsin 53188-1696 (US)
(72) Inventor: Ohe, Yasuhiro, Hino-Shi, Tokyo 191-8503 (JP); Kosugi, Susumu, Hino-Shi, Tokyo 191-8503 (JP); Miyoshi, Mitsuharu, Hino-Shi, Tokyo 191-8503 (JP)
(74) Representative: Goode, Ian Roy

(57) **Abstract**

Medical software is easily evaluated, used and updated on a medical image diagnostic device. To simply evaluate or purchase an option part, an MRI device registering medical software with use limitation is connected by a network to a vendor management server device managed by a vendor as a provider of the medical software. When the "use" request button or the "purchase" request button of the medical software is clicked (a28) on a customer Web Page screen, the MRI device sends (a29) the use request or the purchase request through the network to the vendor management server device. Upon reception (s21) of the use request or the purchase request, the vendor management server device sends (s24) an option key for making the medical software available through the network to the MRI device.

## Description

The present invention relates to a medical software providing method, a method for transacting an option part for a medical image diagnostic device, a medical software providing system, and a system for transacting an option part for a medical image diagnostic device. More specifically, the present invention relates to a medical software providing method which can easily evaluate, use, and update medical software on a medical image diagnostic device, a method for transacting an option part for a medical image diagnostic device which can simply evaluate and purchase the option part, a medical software providing system which executes the medical software providing method, and a system for transacting an option part for a medical image diagnostic device which executes the method for transacting an option part for a medical image diagnostic device.

In general, a sophisticated medical image diagnostic device needs to add and update medical software or an option part to cope with development or improvement in an imaging technique or an image generation algorithm. For example, an MRI (Magnetic Resonance Imaging) device needs to add and purchase medical software or RF coil (Radio Frequency Coil) to image a new Pulse Sequence Design.

However, the medical software and the option part are expensive. Customers have evaluated the quality of an image exhibited by a vendor demonstrated in an exhibition, an image shown by a sales person in charge, or an image obtained by a medical image diagnostic device being operated in another hospital, and then, judged whether the medical software or the option part should be purchased or not.

When a decision is made to purchase new medical software or a new option part, the decision to purchase it is notified to a sales person in charge so as to have it delivered. In other words, when the medical software is purchased, the customer has a service person visited to install the medical software recorded onto an FD (Floppy Disk) on the medical image diagnostic device. Similar installation is also necessary to update the medical software to a newest version or bug-correction version. Further, the option part is purchased, the customer has the option part delivered.

As in the prior art, when an image obtained by other than the medical image diagnostic device of a customer is evaluated, the evaluation is difficult to make due to the difference in system environments (device types and install conditions).

Each time medical software is purchased or updated, it is necessary to ask the service person to perform the installation. This takes the customer a lot of trouble and time.

Once the installation has been completed, the medical software can be used without limitation. It is difficult to sufficiently meet trail use for evaluating the medical software or the needs of a customer who temporarily uses it.

When the option part is evaluated or purchased, the wish to lend the option part or the decision to purchase it must be notified to the sales person in charge. The procedure can be troublesome.

Therefore, a first object of the present invention is to provide a medical software providing method and system which can easily evaluate, use and update medical software on a medical image diagnostic device.

A second object of the present invention is to provide a method and system for transacting an option part for a medical image diagnostic device which can simply evaluate or purchase the option part.

In a first aspect, the present invention provides a medical software providing method comprising the steps of: connecting, by a network, a medical image diagnostic device to a vendor management server device managed by a vendor as a provider of medical software; registering medical software with use limitation in the medical image diagnostic device; upon request to use the medical software by the medical image diagnostic device, sending the use request through the network to the vendor management server device; and when the vendor management server device receives the use request, sending an option key for making the medical software available to the medical image diagnostic device.

In the medical software providing method of the first aspect, when the medical software is used on the medical image diagnostic device, the use request is sent through the network to the vendor management server device so as to receive the option key from the vendor management server device. The option key is used to make the medical software registered in the medical image diagnostic device available.

When the medical software is used, an option key having a small amount of data is only received through the network so as to reduce the download time as compared with when the medical software itself is received. However, without sending the option key through the network, a portable recording medium recording the option key may be sent to the medical image diagnostic device.

When (or before and after) the option key is sent to the medical image diagnostic device, electronic commerce for charging the customer for the use fee can be realized. The use may be permanent use corresponding to "purchase" or temporary use corresponding to "use" as needed.

When "use limitation" of the medical software limits, for example, the number of startups, the use period, or the number of scans, an opportunity to try the medical software in the system environment of the customer can be provided.

In addition, when the "use limitation" is "non-executable", it is possible to inhibit the customer from using the medical software without permission.

In a second aspect, the present invention provides a medical software providing method comprising the steps of: connecting, by a network, a medical image diagnostic device to a vendor management server device managed by a vendor as a provider of medical software; registering medical software with use limitation in the medical image diagnostic device; upon request to use the medical software on a customer Web Page displayed on the medical image diagnostic device, sending the use request through the network to the vendor management server device; and when the vendor management server device receives the use request, sending an option key for making the medical software available to the medical image diagnostic device.

The medical software providing method of the second aspect has the same effect as that of the medical software providing method in the first aspect. While the customer browses the customer Web Page screen displaced on the medical image diagnostic device, the medical software specification or the request instruction can be done exactly under a unified GUI (Graphical User Interface) environment. Since, in general, the contents of the Web Page can be easily changed or added, the vendor manage sever device can readily perform a process for correcting the contents of the customer Web Page in accordance with the current state of the customer.

In a third aspect, the present invention provides a medical software providing method comprising the steps of: connecting, by a network, a medical image diagnostic device to a vendor management server device managed by a vendor as a provider of medical software; registering medical software in the medical image diagnostic device; upon request to update the medical software on a customer Web Page displayed on the medical image diagnostic device, sending the update request through the network to the vendor management server device; and when the vendor management server device receives the update request, sending updating data of the medical software to the medical image diagnostic device.

In the medical software providing method of the third aspect, when the medical software registered in the medical image diagnostic device is updated, the update request is sent through the network to the vendor mange server device to receive the updating data from the vendor management server device. The updating data is used to update the medical software.

The trouble and time of the customer required for update can be saved. When (or before and after) the updating data is sent to the medical image diagnostic device, electronic commerce for charging the customer for the update fee can be realized.

In a fourth aspect, the present invention provides the medical software providing method thus constructed, wherein the vendor management server device charges a customer or a payment organization thereof corresponding to the medical image diagnostic device for a use or update fee of the medical software, and sends the option key or the updating data to the medical image diagnostic device only when the charging is successful.

In the medical software providing method of the fourth aspect, only when use or update of the medical software is charged successfully, the transaction with the customer is responded. Electronic commerce for reliably charging the fee can be realized.

In a fifth aspect, the present invention provides the medical software providing method thus constructed, wherein the vendor management server device sends the medical software through the network to the medical image diagnostic device, and registers the medical software in the medical image diagnostic device.

In the medical software providing method of the fifth aspect, the medical software is registered through the network in the medical image diagnostic device. It is possible to save the trouble and time for passing a portable recording medium recording the medical software to the customer.

In a sixth aspect, the present invention provides the medical software providing method thus constructed, wherein the vendor management server device sends the customer Web Page through the network to the medical image diagnostic device, and registers the customer Web Page to the medical image diagnostic device.

In the medical software providing method of the sixth aspect, the customer Web Page is registered through the network in the medical image diagnostic device. It is possible to save the trouble and time for passing a portable recording medium recording the customer Web Page to the customer.

In a seventh aspect, the present invention provides the medical software providing method thus constructed, wherein the medical image diagnostic device reads and displays through the network the customer Web Page intensively managed by the vendor management server device.

In the medical software providing method of the seventh aspect, the medical image diagnostic device reads and displays through the network the customer Web Page intensively managed by the vendor management server device. The customer Web Page does not need to be registered in the medical image diagnostic device so as to save the storage capacity of the medical image diagnostic device. When the contents of the customer Web Page are corrected in accordance with the current state of the customer, the latest customer Web Page can always be browsed.

In an eighth aspect, the present invention provides the medical software providing method thus constructed, wherein the medical image diagnostic device is at least one of an MRI device, an X-ray CT (Computed Tomography) device, an ultrasound diagnostic device, a PET (Positron Emission Computed Tomography) device, and a digitized X ray (digital X-ray imaging and X-ray film digitization) and CR (Computed Radiography) device.

In the medical software providing method of the eighth aspect, it is possible to preferably provide medical software for use in various medical image diagnostic devices (MRI device, X-ray CT device, ultrasound diagnostic device, PET device, and digitized X-ray and CR device).

In a ninth aspect, the present invention provides a method for transacting an option part for a medical image diagnostic device comprising the steps of: connecting, by a network, a medical image diagnostic device to a vendor management server device managed by a vendor as a provider of an option part for use in the medical image diagnostic device; upon request to lend or purchase the option part on a customer Web Page displayed on the medical image diagnostic device, sending a transaction request through the network to the vendor management server device; and responding to the lending or the purchase by the vendor when the vendor management server device receives the transaction request.

In the method for transacting an option part for a medical image diagnostic device of the ninth aspect, the customer only instructs to lend or purchase the option part on the customer Web Page screen displayed on the medical image diagnostic device. Without any complicated procedure, the option part can be borrowed for evaluation or purchased from the vendor side.

In a tenth aspect, the present invention provides a medical software providing system comprising: a medical image diagnostic device registering medical software with use limitation; a vendor management server device managed by a vendor as a provider of the medical software; and a network connecting the medical image diagnostic device to the vendor management server device, wherein upon request to use the medical software, the medical image diagnostic device sends the use request through the network to the vendor management server device, and wherein upon reception of the use request, the vendor management server device sends an option key for making the medical software available to the medical image diagnostic device.

The medical software providing system of the tenth aspect can preferably execute the medical software providing method of the first aspect.

In an eleventh aspect, the present invention provides a medical software providing system comprising: a medical image diagnostic device registering medical software with use limitation; a vendor management server device managed by a vendor as a provider of the medical software; and a network connecting the medical image diagnostic device to the vendor management server device, wherein upon request to use the medical software on a customer Web Page, the medical image diagnostic device sends the use request through the network to the vendor management server device, and wherein upon reception of the use request, the vendor management server device sends an option key for making the medical software available to the medical image diagnostic device.

The medical software providing system of the eleventh aspect can preferably execute the medical software providing method of the second aspect.

In a twelfth aspect, the present invention provides a medical software providing system comprising: a medical image diagnostic device registering medical software; a vendor management server device managed by a vendor as a provider of the medical software; and a network connecting the medical image diagnostic device to the vendor management server device, wherein upon request to update the medical software on a customer Web Page, the medical image diagnostic device sends the update request through the network to the vendor management server device, and wherein upon reception of the update request, the vendor management server device sends updating data of the medical software to the medical image diagnostic device.

The medical software providing system of the twelfth aspect can preferably execute the medical software providing method of the third aspect.

In a thirteenth aspect, the present invention provides the medical software providing system thus constructed, wherein the vendor management server device charges a customer or a payment organization thereof corresponding to the medical image diagnostic device for a use or update fee of the medical software, and sends the option key or the updating data to the medical image diagnostic device only when the charging is successful.

The medical software providing system of the thirteenth aspect can preferably execute the medical software providing method of the fourth aspect.

In a fourteenth aspect, the present invention provides the medical software providing system thus constructed, wherein the vendor management server device sends the medical software through the network to the medical image diagnostic device, and registers the medical software in the medical image diagnostic device.

The medical software providing system of the fourteenth aspect can preferably execute the medical software providing method of the fifth aspect.

In a fifteenth aspect, the present invention provides the medical software providing system thus constructed, wherein the vendor management server device sends the customer Web Page through the network to the medical image diagnostic device, and registers the customer Web Page to the medical image diagnostic device.

The medical software providing system of the fifteenth aspect can preferably execute the medical software providing method of the sixth aspect.

In a sixteenth aspect, the present invention provides the medical software providing system thus constructed, wherein the medical image diagnostic device reads and displays through the network the customer Web Page intensively managed by the vendor management server device.

The medical software providing system of the sixteenth aspect can preferably execute the medical software providing method of the seventh aspect.

In a seventeenth aspect, the present invention provides the medical software providing system thus constructed, wherein the medical image diagnostic device is at least one of an MRI device, an X-ray CT device, an ultrasound diagnostic device, a PET device, and a digitized X-ray and CR device.

The medical software providing system of the seventeenth aspect can preferably execute the medical software providing method of the eighth aspect.

In an eighteenth aspect, the present invention provides a system for transacting an option part for a medical image diagnostic device comprising: a medical image diagnostic device; a vendor management server device managed by a vendor as a provider of an option part for use in the medical image diagnostic device; and a network connecting the medical image diagnostic device to the vendor management server device, wherein upon request to lend or purchase the option part on a customer Web Page, the medical image diagnostic device sends a transaction request through the network to the vendor management server device, and wherein upon reception of the transaction request, the vendor management server device sends a transaction establishment notice through the network to the medical image diagnostic device.

The system for transacting an option part for a medical image diagnostic device of the eighteenth aspect can suitably execute the method for transacting an option part for a medical image diagnostic device of the ninth aspect.

In the medical software providing method and system according to the present invention, the customer only instructs to purchase or update the medical software on the medical image diagnostic device (such as the MRI device, X-ray CT device, and the ultrasound diagnostic device) to receive the option key for releasing use limitation or updating data from the vendor side. The latest medical software can be used easily and rapidly. In particular, when the customer instruction is received on the customer Web Page screen corrected corresponding to the current state of the customer, the customer can efficiently perform electronic commerce under the unified GUI environment, and the usability is high.

In the method and system for transacting an option part for a medical image diagnostic device according to the present invention, the customer only instructs to lend or purchase the option part on the customer Web Page screen displayed on the medical image diagnostic device to borrow for evaluation or purchase the option part from the vendor side without any troublesome procedure.

Further objects and advantages of the present invention will be apparent from the following description of the preferred embodiments of the invention as illustrated in the accompanying drawings, in which:
FIG. 1 is a block diagram showing a medical software providing system according to the present invention;
FIG. 2 is a block diagram of a vendor management server device and an MRI device in the medical software providing system of FIG. 1;
FIG. 3 is a flowchart showing a procedure for registering a customer Web Page and medical software in a medical image diagnostic device and updating the register contents;
FIG. 4 is a flowchart showing a procedure for evaluating/using/purchasing the medical software on the medical image diagnostic device;
FIG. 5 is an illustration of a customer Web Page screen;
FIG. 6 is an illustration of a truing message screen;
FIG. 7 is an illustration of an error message screen;
FIG. 8 is an illustration of a customer Web Page screen according to a second embodiment;
FIG. 9 is a block diagram of a vendor management server device and an MRI device according to a third embodiment;
FIG. 10 is a flowchart showing a procedure in which a customer requests to lend/purchase an option part;
FIG. 11 is another illustration of the customer Web Page screen; and
FIG. 12 is an illustration of a purchase confirmation message screen.

The present invention will be described in detail hereinbelow by the embodiments illustrated.

### First Embodiment

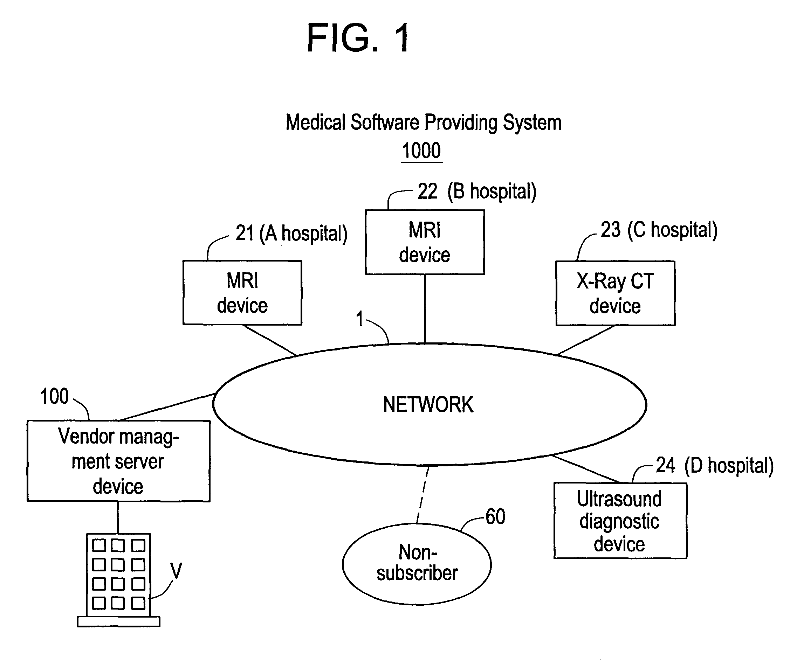

FIG. 1 is block diagram showing a medical software providing system 1000 according to a first embodiment of the present invention.

The medical software providing system 1000 has a network 1 such as Internet, LAN (Local Area Network), and WAN (Wide Area Network), an MRI device 21 connected to the network 1 (installed in A hospital), an MRI device 22 (installed in B hospital), an X-ray CT device 23 (installed in C hospital), an ultrasound diagnostic device 24 (installed in D hospital), and a vendor management server device 100 managed by a Vendor V. The network 1 may be connected to a PET device or a digitized X-ray and CR device.

When the network is Internet, a non-subscriber 60 with a terminal not contracting use of the vendor management server device 100 is also connected to the network 1.

In view of security, SSL (Secure Socket Layer Protocol) and so on is preferably used.

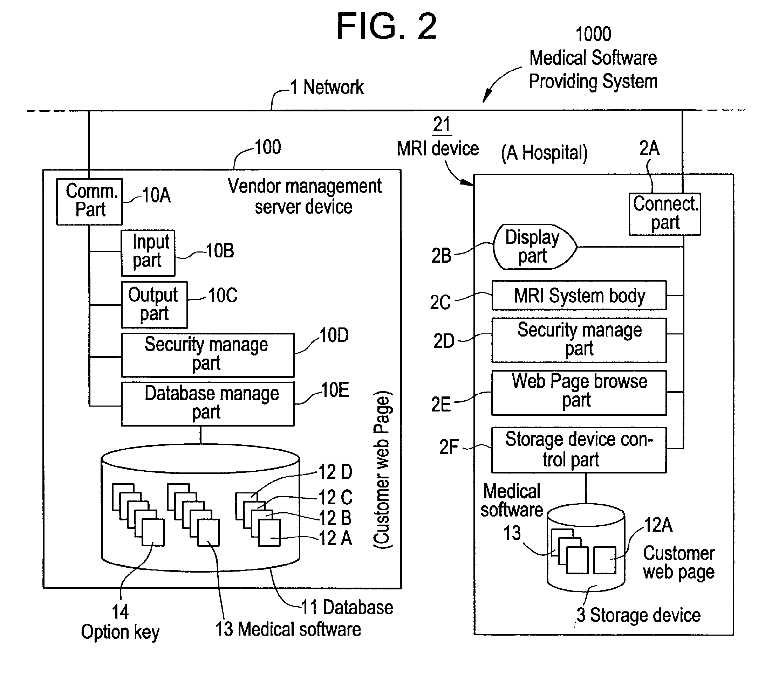

Customers (A to D hospitals) operating the MRI devices 21 and 22, the X-ray CT device 23, and the ultrasound diagnostic device 24 are respectively contracted as a use subscriber of the vendor management server device 100. A subscriber program recorded onto a recording medium (such as CD-ROM or FD) distributed from the Vendor V or a subscriber program distributed through the network 1 is executed on the respective devices to function as the use subscriber. The medical software or the customer Web Page is received through the network 1 so as to be registered in a local storage device (the numeral 3 of FIG. 2).

The vendor management server device 100 holds subscriber information.
The subscriber information is created and updated as follows.
(1) A new subscriber concludes a contract to receive the medical software provision with the Vendor V, and receives a recording medium storing a program corresponding to the contract contents. Otherwise, the new subscriber downloads the program through the network 1;
(2) When the new subscriber installs the program and starts up it for the first time, update of the subscriber information is automatically requested through the network 1 to the vendor management server device 100. Then, the vendor management server device 100 adds the new subscriber to the subscriber information; and
(3) Based on cancel of the contract of the subscriber, the vendor management server device 100 deletes the subscriber from the subscriber information.

The Vendor V receives the subscribing fee on contract. The Vender V also receives the manage fee for a service for maintaining and updating the subscriber information at fixed or unfixed intervals. The Vendor V sells the program as well.

FIG. 2 is a block diagram of the vendor management server device 100 and the MRI device 21.

The vendor management server device 100 has a communication part 10A, an input part 10B, an output part 10C, a security manage part 10D, a database manage part 10E, and a database 11, and is operated under control of the medical information manage program.

The database 11 stores a Web Page for each customer, that is, a customer Web Page 12A corresponding to the MRI device 21, a customer Web Page 12B corresponding to the MRI device 22, a customer Web Page 12C corresponding to the X-ray CT device 23, and a customer Web Page 12D corresponding to the ultrasound diagnostic device 24. The database 11 also stores a large number of pieces of medical software 13 for use in the medical image diagnostic device such as the MRI device. The medical software 13 incorporates a function limiting the use thereof. The database 11 further stores an option key 14 for making the medial software 13 available (releasing the use limitation). The recording medium of the database 11 is, for example, a large-volume hard disk.

The MRI device 21 has a connection part 2A, a display part 2B provided with a CRT (Cathode Ray Tube) or an LCD (Liquid Crystal Display), an MRI system body 2C, a security manage part 2D, a Web Page browse part 2E, a storage device control part 2F, and a storage device 3 such as a hard disk. The storage device 3 registers the customer Web Page 12A and the medical software 13 sent from the vendor management server device 100 (details will be described later). The connection part 2A is formed by executing the connection program for communication through the network 1. The Web Page browse part 2E is formed by executing a Web Browser such as UNIX-version Netscape Navigator (manufactured by Netscape Communications Corporation).

The X-ray CT device 23 has an X-ray CT system body in place of the MRI system body 2C. The ultrasound diagnostic device 24 has an ultrasound diagnostic system body in place of the MRI system body 2C.

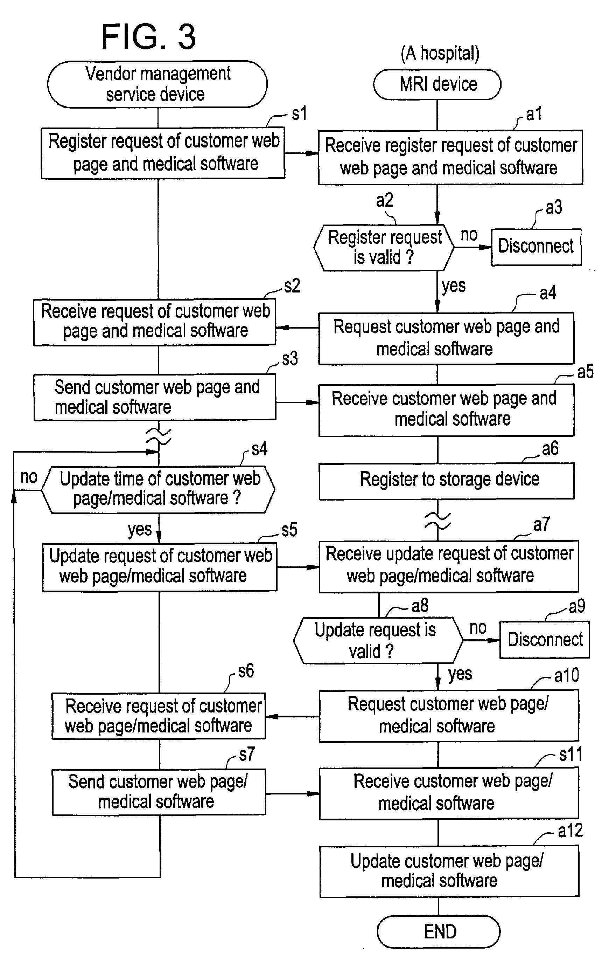

FIG. 3 is a flowchart showing a procedure for registering the customer Web Page (the numeral 12A in this example) and the medical software 13 in the medical image diagnostic device and updating the contents registered. The left side flow is a flow of the vendor management server device 100. The right side flow is a flow of the medical image diagnostic device (envisaging the MRI device 21).

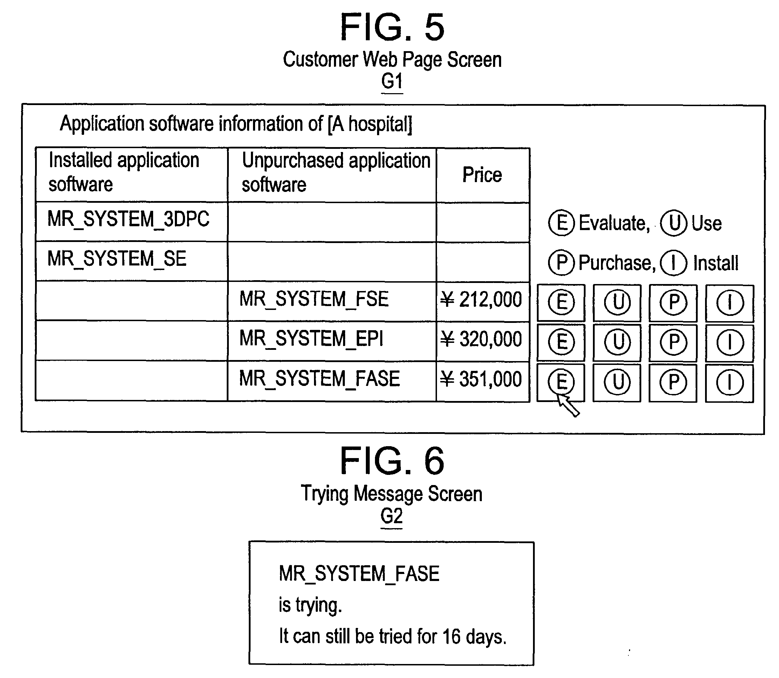

In step s1, the vender manage server device 100 requests the MRI device 21 through the network 1 to register the customer Web Page 12A and the medical software 13. The specific examples of the customer Web Page 12A and the medical software 13 will be described later in detail with reference to FIG. 5.

In step a1, the MRI device receives the register request.

In step a2, the security manage part 2D of the MRI device 21 checks validity of the register request. When it is invalid, the routine is advanced to step a3. When it is valid, the routine is advanced to step a4. To check this validity, a known validity check method for connecting communication lines such as check of a network address or telephone number, check of a password, or check of an ID card is used.

In step a3, the communication line is disconnected.

In step a4, the customer Web Page 12A and the medical software 13 are requested to the vendor management server device 100.

In step s2, the vendor management server device 100 receives the request of the customer Web Page 12A and the medical software 13.

In step s3, the customer Web Page 12A and the medical software 13 are sent through the network 1 to the MRI device 21.

In step a5, the MRI device 21 receives the customer Web Page 12A and the medical software 13.

In step a6, the customer Web Page 12A and the medical software 13 are registered in the storage device 3.

In step s4, the vendor management server device 100 waits for the time of update of the customer Web Page 12A and/or the medical software 13. At the time of update, the routine is advanced to step s5. The customer Web Page 12A and/or the medical software 13 is updated when a fixed period elapses from register or the previous update, or when the contents of the customer Web Page 12A or the medical software 13 are modified.

In step s5, the update request of the customer Web Page 12A and/or the medical software 13 is sent through the network 1 to the MRI device 21. In step a7, the MRI device 21 receives the update request.

In step a8, the security manage part 2D of the MRI device 21 checks validity of the update request. When it is invalid, the routine is advanced to step a9. When it is valid, the routine is advanced to step a10.

In step a9, the communication line is disconnected.

In step a10, the customer Web Page 12A and the medical software 13 are requested through the network 1 to the vendor management server device 100.

In step s6, the vendor management server device 100 receives the request of the customer Web Page 12A and/or the medical software 13.

In step s7, the newest customer Web Page 12A and/or medical software 13 are sent through the network 1 to the MRI device 21.

In step a11, the MRI device 21 receives the customer Web Page 12A and/or the medical software 13.

In step a12, the register contents of the customer Web Page 12A and/or the medical software 13 are updated.

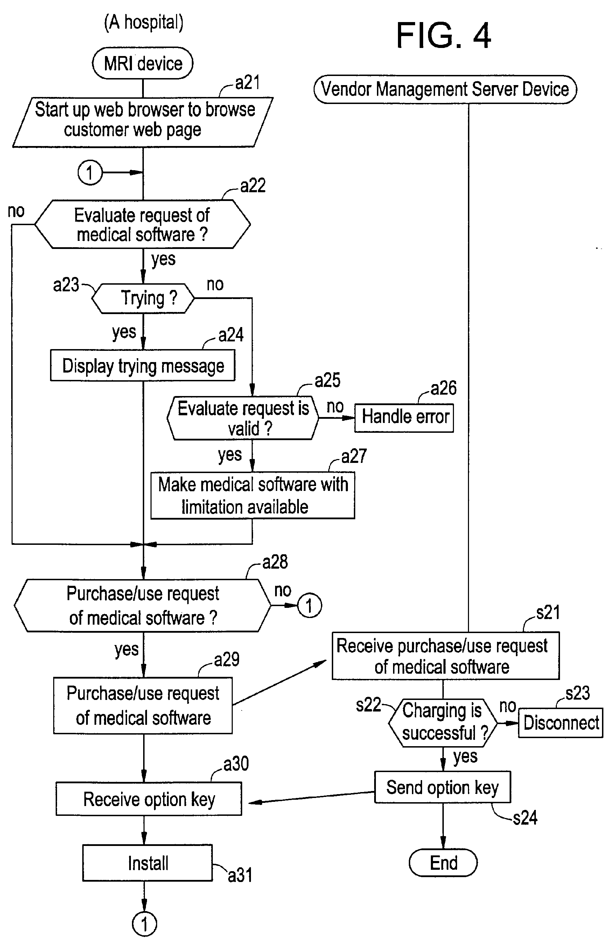

FIG. 4 is a flowchart showing a procedure for evaluating/using/purchasing the medical software 13 on the medical image diagnostic device. The left side flow is a flow of the medical image diagnostic device (envisaging the MRI device 21). The right side flow is a flow of the vendor management server device 100.

In step a21, the customer operating the MRI device 21 starts up the Web Browser to browse the customer Web Page 12A. For example, as shown in the customer Web Page G1 of FIG. 5, as application software information on [A hospital], a listing of installed application software, unpurchased application software, and prices thereof is displayed. The "evaluate" request button, the "use" request button, the "purchase" request button, and the "install" button are also displayed corresponding to each piece of the unpurchased application software. The "evaluate" means that the medical software 13 is tried with limitation. The "use" means that the right to use the medical software 13 once or for a fixed period of time is obtained by paying the fee as necessary. The "purchase" means that the medical software 13 is purchased (generally, the permanent license agreement is concluded). The "install" means that the option key 14 for making the medical software 13 available is fetched into the system.

On the illustrated customer Web Page screen G1, [MR_SYSTEM_3DPC] is software of 3DPC (Dimension Phase Collection) as one kind of the MRI imaging option. [MR_SYSTEM_SE] is pulse sequence software of an SE (Spin Echo) method. [MR_SYSTEM_FSE] is pulse sequence software of an FSE (Fast Spin Echo) method. [MR_SYSTEM_EPI] is pulse sequence software an EPI (Echo Planar Imaging) method. [MR_SYSTEM_FASE] is pulse sequence software of FASE (Fast Asymmetric Spin Echo) method.

In step a22, on the customer Web Page screen G1 displayed on the MRI device 21, when the "evaluate" request button to any one piece of the unpurchased application software is clicked, the routine is advanced to step a23. Otherwise, the routine is advanced to step a28.

In step a23, when the medical software 13 requested for evaluation is being tried, the routine is advanced to step a24. When it is not being tried, the routine is advanced to step a25.

In step a24, the trying message is displayed. For example, as shown in the trying message screen G2 of FIG. 6, the message "MR_SYSTEM_FASE" is trying. It can still be tried for 16 days." is displayed.

In step a25, the validity of the evaluation request is checked. When it is invalid, the routine is advanced to step a26, and when it is valid, the routine is advanced to step a27. For example, when the previously tried medical software 13 is requested for evaluation again, it is judged to be invalid.

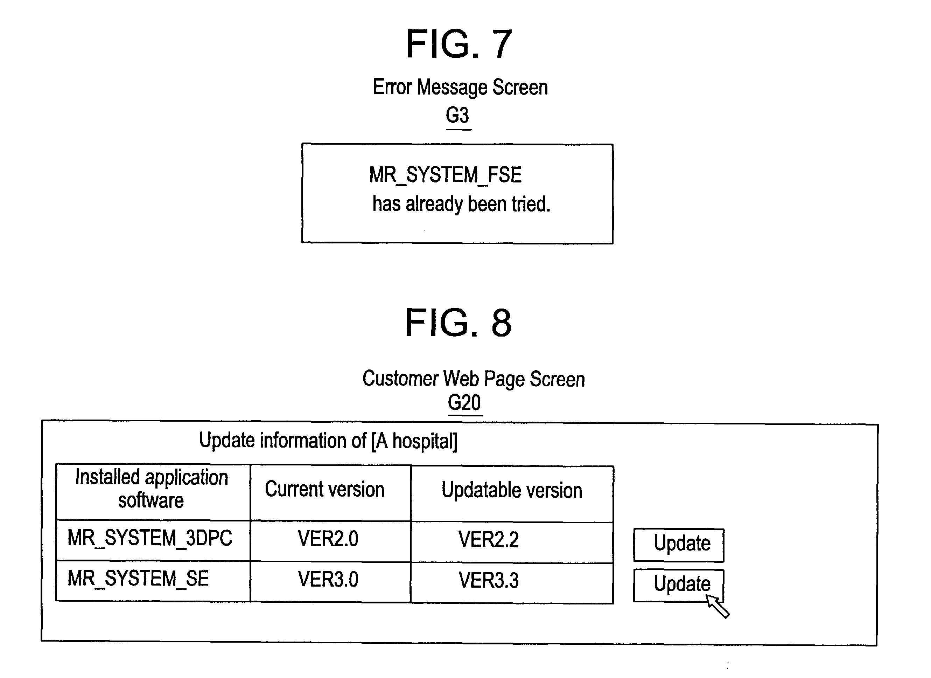

In step a26, errors are handled. For example, as shown in the error message screen G3 of FIG. 7, the message "MR_SYSTEM_FSE has already been used." is displayed.

In step a27, the medical software 13 can be used with limitation. The limitation limits the number of startups, the use period, or the number of scans.

In step a28, the "purchase" request button or the "use" request button to any one piece of the unpurcahsed application software is clicked, the routine is advanced to step a29. Otherwise, the routine is returned to the step a22.

In step a29, the purchase request or the use request of the unpurchased application software specified in the step a28 is sent through the network 1 to the vendor management server device 100.

In step s21, the vendor management server device 100 receives the purchase request or the use request.

In step s22, the purchase fee or the use fee is charged to the customer or the payment organization thereof. When the charging is successful, the routine is advanced to step s24. Otherwise, the routine is advanced to step s23. For example, the fee charge screen, not shown, is displayed on the display part 2B of the MRI device 21 to charge the purchase fee or the use fee. When the customer operates to pay the fee by transferring money from the bank account or by a credit card or a payment agent service, the charging is judged to be successful.

In step s23, the communication line is disconnected.

In step s24, the option key 14 for making the corresponding medical software 13 available is sent through the network 1 to the MRI device 21.

In other words, in the case of "purchase", the option key 14 for permanently making the medical software 13 available is sent.

In the case of "use", the option key 14 for temporarily making the medical software 13 available is sent. For example, the option key 14 is sent to limit the number of startups or the number of scans (which may be one or more) and to limit the use period (which may be one day or longer and shorter).

In step a30, the MRI device 21 receives the option key 14.

In step a31, the "install" button (see FIG. 5) corresponding to the corresponding medical software 13 is clicked to install the option key 14. This makes the medical software 13 available.

According to the medical software providing system 1000 of the first embodiment, the medical software 13 can be tried in the system environment of the customer to correctly evaluate whether the medical software 13 can be purchased or not. When the medical software 13 is properly purchased or used, the option key 14 having a small amount of data is only received through the network 1 to execute the medical software 13 rapidly.

A CD-ROM storing the medical software 13 with use limitation may be passed to the customer, and then, the customer may read the medical software 13 from the medium to register it in the medical image diagnostic device. In this case, the medical software 13 having a large amount of data does not need to be transferred through the network 1. The register operation can be completed for a short time.

The customer Web Page (the numeral 12A in the above example) intensively managed by the vendor management server device 100 may be browsed through the network 1. In this case, the customer Web Page does not need to be registered in the storage device 3 of the medical image diagnostic device to save the volume of the storage device 3.

Without transmitting the option key 14 through the network 1, an FD recording the option key 14 may be passed to the customer side. In this case, the possibility that the option key 14 may be incorrectly obtained, duplicated, or changed can be reduced to further improve the security.

### Second Embodiment

In the second embodiment, the display part of the medical image diagnostic device displays the customer Web Page screen for updating the installed application software. For example, as shown in the customer Web Page screen G20 of FIG. 8, as the update information on [A hospital], the current version, the updateable version, and the "update" request button are displayed for each piece of installed application software. In the illustrated example, [MR_SYSTEM_3DPC] can be updated from the current version 2.0 to the version 2.2. [MR_SYSTEM_SE] can be updated from the current version 3.0 to the version 3.3. The customer Web Page as a base of the screen is registered in the storage device 3 of the medical image diagnostic device of the customer side by the procedure explained previously with reference to FIG. 3.

When the customer clicks the "update" request button corresponding to the installed application software to be updated, the update request is sent through the network 1 to the vendor management server device 100. Then, the vendor management server device 100 sends updating data of the new version through the network 1 to the corresponding medical image diagnostic device. The installed application software is thus updated to the newest version.

According to the second embodiment, when the customer only clicks the "update" request button of the customer Web Page screen G20 displayed on the medical image diagnostic device, the customer receives the updating data from the vendor management server device 100 to update the installed application software.

In the second embodiment, instead of or adding version up of the installed application software, the installed application software may be updated to a bug correction version.

### Third Embodiment

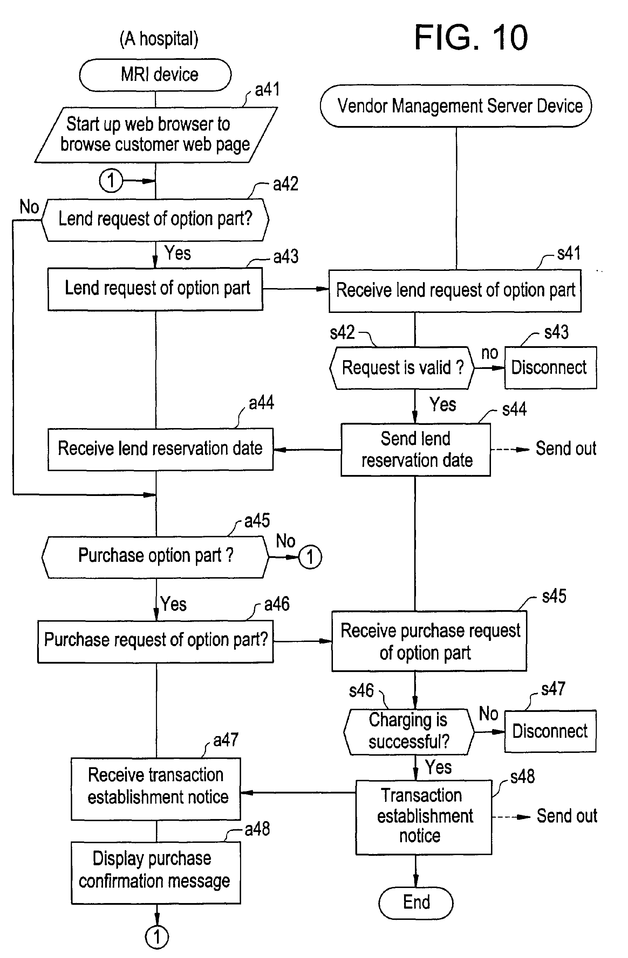

In a third embodiment, the display device of the medical image diagnostic system of the customer side displays the customer Web Page screen (the numeral G30 of FIG. 10) for transacting an option part to transact the option part.

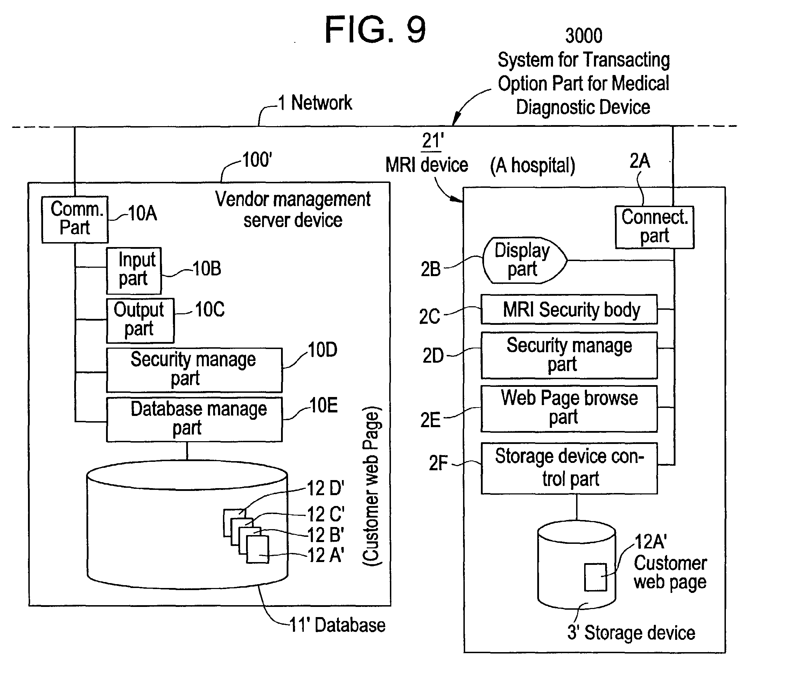

FIG. 9 is a block diagram of a vendor management server device 100' and an MRI device 21' of a system 3000 for transacting an option part for a medical image diagnostic device according to the third embodiment.

A database 11' of the vendor management server device 100' stores customer Web Pages 12A', 12B', 12C' and 12D' for each customer.

The storage device 3 of the MRI device 21' registers the customer Web Page 12A' (the registering method is as explained previously with reference to FIG. 3.).

FIG. 10 is a flowchart showing a procedure in which the customer requests to lend or purchase an option part. The left side flow is a flow of the medical image diagnostic device (envisaging the MRI device 21'). The right side flow is a flow of the vendor management server device 100.

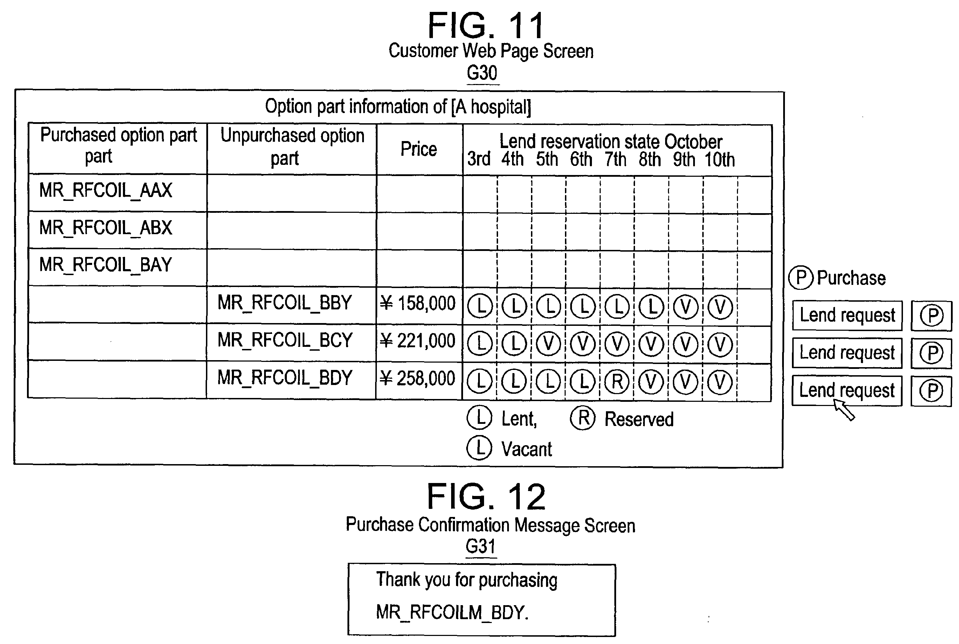

In step a41, the customer operating the MRI device 21' starts up the Web Browser to browse the customer Web Page. For example, as shown in the customer Web Page screen G30 of FIG. 11, as the option part information on [A hospital], a listing of (the model name) of purchased option parts, unpurchased option parts, prices thereof, and lend reservation states thereof is displayed. The "lend request" button and the "purchase" request button are also displayed corresponding to each of the unpurchased option parts. In displaying the schedule of the lend reservation states, the "lent" means that other customer has reserved the unpurchased option part, the "reserved" means that A hospital has reserved it, and the "vacant" means that it can be lent. The option part is, for example, an RF coil for MRI.

In step a42, the "lend request" button to any one of the unpurchased option parts is clicked on the customer Web Page screen G30 displayed on the MRI device 21', the routine is advanced to step a43. Otherwise, the routine is advanced to step a45.

In step a43, the lend request of the unpurchased option part is sent through the network 1 to the vendor mange server device 100.

In step s41, the vendor management server device 100 receives the lend request.

In step s42, the validity of the lend request is checked. When it is invalid, the routine is advanced to step s43, and when it is valid, the routine is advanced to step s44. For example, when the same unpurcahsed option part has previously been lent, it is judged to be invalid.

In step s43, the communication line is disconnected.

In step s44, the lend reservation date is decided, and the then lend reservation date is sent through the network 1 to the MRI device 21'. Thereafter, the vendor sends out the unpurchased option part to the customer on the lend reservation date.

In step a44, the MRI device 21' receives the lend reservation date. The lend reservation date is reflected to the customer Web Page screen G30 (see FIG. 11).

In step a45, when the "purchase" request button to any one of the unpurchased option parts is clicked, the routine is advanced to step a46. Otherwise, the routine is returned to the step a42.

In step a46, the purchase request to the unpurcahsed option part is sent through the network 1 to the vendor management server device 100.

In step s45, the vendor management server device 100 receives the purchase request.

In step s46, the purchase fee is charged to the customer or the payment organization thereof. When the charging is successful, the routine is advanced to step s48. Otherwise, the routine is advanced to step s47.

In step s47, the communication line is disconnected.

In step s48, a transaction establishment notice is sent through the network 1 to the MRI device 21'. The transaction establishment is notified to the sales department, the production department, and the service department of the vendor. The sales department sends out an order form to the customer. The production department sends out an option part to the customer. The service department updates the customer Web Page 12A to the newest contents to update the register contents of the MRI device 21'.

In step a47, the MRI device 21' receives the transaction establishment notice.

In step a48, the purchase confirmation message is displayed. For example, as shown in the purchase confirmation message screen G31 of FIG. 12, the message "Thank you for purchasing MR_RFCOIL_BDY." is displayed. Thereafter, the routine is returned to the step a42.

According to the third embodiment, the customer only clicks the "lend request" button or the "purchase" request button on the customer Web Page screen G30 displayed on the medical image diagnostic device (e.g., the MRI device 21') to borrow the option part from the vendor side for evaluation or to purchase it.

## Claims

1. A medical software providing method comprising the steps of:
connecting, by a network, a medical image diagnostic device to a vendor management server device managed by a vendor as a provider of medical software;
registering medical software with use limitation in the medical image diagnostic device;
upon request to use the medical software by the medical image diagnostic device, sending the use request through the network to the vendor management server device; and
when the vendor management server device receives the use request, sending an option key for making the medical software available to the medical image diagnostic device.

2. A medical software providing method comprising the steps of:
connecting, by a network, a medical image diagnostic device to a vendor management server device managed by a vendor as a provider of medical software;
registering medical software with use limitation in the medical image diagnostic device;
upon request to use the medical software on a customer Web Page displayed on the medical image diagnostic device, sending the use request through the network to the vendor management server device; and
when the vendor management server device receives the use request, sending an option key for making the medical software available to the medical image diagnostic device.

3. A medical software providing method comprising the steps of:
connecting, by a network, a medical image diagnostic device to a vendor management server device managed by a vendor as a provider of medical software;
registering medical software in the medical image diagnostic device;
upon request to update the medical software on a customer Web Page displayed on the medical image diagnostic device, sending the update request through the network to the vendor management server device; and
when the vendor management server device receives the update request, sending updating data of the medical software to the medical image diagnostic device.

4. The medical software providing method according to any one of claims 1 to 3, wherein the vendor management server device charges a customer or a payment organization thereof corresponding to the medical image diagnostic device for a use or update fee of the medical software, and sends the option key or the updating data to the medical image diagnostic device only when the charging is successful.

5. The medical software providing method according to any one of claims 1 to 3, wherein the vendor management server device sends the medical software through the network to the medical image diagnostic device, and registers the medical software in the medical image diagnostic device.

6. method for transacting an option part for a medical image diagnostic device comprising the steps of:
connecting, by a network, a medical image diagnostic device to a vendor management server device managed by a vendor as a provider of an option part for use in the medical image diagnostic device;
upon request to lend or purchase the option part on a customer Web Page displayed on the medical image diagnostic device, sending a transaction request through the network to the vendor management server device; and
responding to the lending or the purchase by the vendor when the vendor management server device receives the transaction request.

7. A medical software providing system comprising:
a medical image diagnostic device registering medical software with use limitation;
a vendor management server device managed by a vendor as a provider of the medical software; and
a network connecting the medical image diagnostic device to the vendor management server device, wherein
upon request to use the medical software, the medical image diagnostic device sends the use request through the network to the vendor management server device, and wherein
upon reception of the use request, the vendor management server device sends an option key for making the medical software available to the medical image diagnostic device.

8. A medical software providing system comprising:
a medical image diagnostic device registering medical software with use limitation;
a vendor management server device managed by a vendor as a provider of the medical software; and
a network connecting the medical image diagnostic device to the vendor management server device, wherein
upon request to use the medical software on a customer Web Page, the medical image diagnostic device sends the use request through the network to the vendor management server device, and wherein
upon reception of the use request, the vendor management server device sends an option key for making the medical software available to the medical image diagnostic device.

9. A medical software providing system comprising:
a medical image diagnostic device registering medical software;
a vendor management server device managed by a vendor as a provider of the medical software; and
a network connecting the medical image diagnostic device to the vendor management server device, wherein
upon request to update the medical software on a customer Web Page, the medical image diagnostic device sends the update request through the network to the vendor management server device, and wherein
upon reception of the update request, the vendor management server device sends updating data of the medical software to the medical image diagnostic device.

10. A system for transacting an option part for a medical image diagnostic device comprising:
a medical image diagnostic device;
a vendor management server device managed by a vendor as a provider of an option part for use in the medical image diagnostic device; and
a network connecting the medical image diagnostic device to the vendor management server device, wherein
upon request to lend or purchase the option part on a customer Web Page, the medical image diagnostic device sends a transaction request through the network to the vendor management server device, and wherein
upon reception of the transaction request, the vendor management server device sends a transaction establishment notice through the network to the medical image diagnostic device.
